# EUROPEAN PATENT APPLICATION

(11) **EP 4 368 182 A1**
(43) Date of publication of application: **15.05.2024**
(21) Application number: 22837088.8
(22) Date of filing: 07.07.2022
(51) Int. Cl.: A61K 31/4985, C07D 471/04, A61P 25/28

(54) **TAU-TUBULIN KINASE (TTBK) INHIBITOR COMPOUNDS**

(30) Priority: 09.07.2021 ES 202130653
(71) Applicant: Consejo Superior De Investigaciones Científicas - CSIC, 28006 Madrid (ES)
(72) Inventor: MARTÍNEZ, Ana, 28006 Madrid (ES); GIL, Carmen, 28006 Madrid (ES); NOZAL, Vanesa, 28006 Madrid (ES); PALOMO, Valle, 28006 Madrid (ES); MARTÍN-REQUERO, Angeles, 28006 Madrid (ES); MARTÍNEZ-GONZÁLEZ, Loreto, 28006 Madrid (ES); PÉREZ CUEVAS, Eva M., 28006 Madrid (ES)
(74) Representative: Pons
(86) International application number: PCT/ES2022/070436
(87) International publication number: WO 2023/281149

(57) **Abstract**

The present invention relates to TTBK enzyme inhibitors of formula (I) and to the use thereof for the treatment and/or prevention of tauopathies, such as Alzheimer's disease (AD), and/or TDP-43 pathologies, such as amyotrophic lateral sclerosis (ALS),

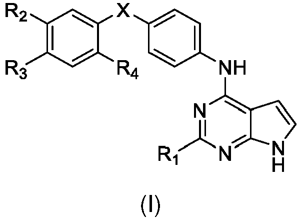

## Description

The present invention relates to TTBK enzyme inhibitors and their use for the treatment and/or prevention of tauopathies and/or TDP-43 diseases. Therefore, the invention may be comprised in the medical chemistry and pharmaceutical field.

### BACKGROUND OF THE INVENTION

Tau-tubulin kinases (TTBK) are a family of recently discovered serine/threonine and tyrosine kinases involved in the phosphorylation of important substrates such as tau, tubulin or TDP-43. Its two isoforms, TTBK1 and TTBK2, show different expression patterns and different participation in highly important physiological mechanisms, such as mitosis, ciliogenesis and neurotransmission.

It has recently been shown that TTBK are also responsible for the phosphorylation of TDP-43 (Liachko NF, et al. The tau tubulin kinases TTBK1/2 promote accumulation of pathological TDP-43. PLoS Genet. 2014 Dec 4;10(12):e1004803), thus their phosphorylation activity has also linked them to the development of neurodegenerative diseases such as Alzheimer's disease and amyotrophic lateral sclerosis. These neurodegenerative diseases are called tauopathies and TDP-43 diseases because they are associated with the pathological aggregation of tau and TDP-43 proteins, respectively, in the human brain.

In the following document: Nozal, A. Martinez, Tau Tubulin Kinase 1 (TTBK1), a new player in the fight against neurodegenerative diseases, Eur J Med Chem 2019 Jan 1;161 :39-47, the importance of the role played by the aforementioned TTBK kinases in neurodegenerative diseases such as Alzheimer's (AD), progressive supranuclear palsy, frontotemporal dementia (FTD), amyotrophic lateral sclerosis (ALS), etc, is already evident, proposing TTBK1 and TTBK2 as new targets for the treatment of said diseases and their selective inhibitors as potential effective drugs for their treatment, having been demonstrated very recently (Taylor LM, McMillan PJ, Liachko NF, Strovas TJ, Ghetti B, Bird TD, Keene CD, Kraemer BC. Pathological phosphorylation of tau and TDP-43 by TTBK1 and TTBK2 drives neurodegeneration. Mol Neurodegener. 2018 Feb 6;13(1):7) which are involved in neurodegenerative processes through not only the hyperphosphorylation of tau but also TDP-43.

Pursuant to the foregoing, inhibition of TTBK may be a promising approach for the treatment of neurodegenerative diseases such as those involving the tau protein and the TDP-43 protein, i.e., tauopathies and TDP-43 diseases.

Given that in the state of the art there are few known compounds that can act as inhibitors of said enzyme, the present invention proposes new compounds that act as inhibitors thereof, being useful for the treatment and/or prevention of neurodegenerative diseases where this enzyme is involved. It is, therefore, an alternative to or improvement on the compounds already known for the treatment of diseases of this type.

### DESCRIPTION OF THE INVENTION

The present invention presents a family of compounds that have the ability to inhibit the TTBK1 and/or TTBK2 enzyme in micromolar order or lower, proving useful for the treatment and/or prevention of tauopathies and/or TDP-43 diseases.

In a first aspect, the present invention relates to a compound of formula (I) 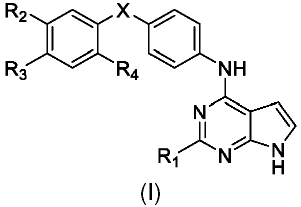 or any of the isomers thereof or the pharmaceutically acceptable salts thereof wherein:
R₁ is selected from H and NH₂,
X is selected from O, S, CO and CH₂O,
R₂, R₃ and R₄ are independently selected from: H, halogen, CF₃, CN, NO₂, NH₂, C₁-C₃ alkyl and -O-C₁-C₃ alkyl,
and wherein at least one of R₂, R₃ and R₄ is H
for use in the treatment and/or prevention of tauopathies and/or TDP-43 diseases.

The compounds of formula (I) act as inhibitors of the TTBK1 and/or TTBK2 enzymes, which are involved in tauopathies and TDP-43 diseases. These are, as indicated in the state of the art section, a class of neurodegenerative diseases associated with the pathological aggregation of tau protein or TDP-43, respectively, in the nervous system. They are, therefore, neurodegenerative diseases where tau-tubulin kinases (TTBK), such as TTBK1 and TTBK2, are involved.

Preferably, tauopathies are selected from the following list: Alzheimer's disease (AD), progressive supranuclear palsy, frontotemporal dementia (FTD), Pick's disease, Down's syndrome. More preferably, the tauopathy is selected from Alzheimer's disease (AD), and frontotemporal dementia (FTD) and, even more preferably, the tauopathy is Alzheimer's disease.

Preferably, the TDP-43 diseases are selected from the following list: amyotrophic lateral sclerosis (ALS), Alexander disease, limbic-predominant age-related TDP-43 encephalopathy (LATE), frontotemporal dementia (FTD) and Huntington's disease. More preferably, the TDP-43 disease is selected from frontotemporal dementia (FTD), limbic-predominant age-related TDP-43 encephalopathy (LATE) and amyotrophic lateral sclerosis (ALS) and, even more preferably, the TDP-43 disease is amyotrophic lateral sclerosis.

In a preferred embodiment of the compounds of the invention for use, at least two of R₂, R₃ and R₄ are H. In another preferred embodiment of the compounds of the invention for use, two of R₂, R₃ and R₄ are H and one of them is different from H.ln another preferred embodiment of the compounds of the invention for use, R₂, R₃ and R₄ are H.

In a preferred embodiment of the compounds of formula (I) for use, R₁ is H.

In another preferred embodiment of the compounds of formula (I) for use, X is O.

In a more preferred embodiment, R₁ is H and X is O. Even more preferably R₁ is H, X is O and R₂, R₃ and R₄ are independently selected from H, halogen, CN, NO₂, NH₂, C₁-C₃ alkyl, -(O)- C₁-C₃ alkyl.

In a preferred embodiment, R₁ is H, X is O, R₂ and R₄ are H and R₃ is selected from H, halogen, CF₃, CN, NO₂, NH₂, C₁-C₃ alkyl, -(O)-C₁-C₃ alkyl, more preferably R₃ is selected from H, halogen, CN, NO₂, NH₂, C₁-C₃ alkyl, -(O)- C₁-C₃ alkyl.

In a preferred embodiment, R₁ is H, X is O, R₂ and R₃ are H and R₄ is selected from halogen and -(O)-C₁-C₃ alkyl.

In a preferred embodiment, R₁ is H, X is O, R₃ and R₄ are H and R₂ is selected from halogen, CF₃, -(O)-C₁-C₃ alkyl and C₁-C₃ alkyl, more preferably, R₂ is selected from halogen, CF₃, -(O)-C₁-C₃ alkyl and alkyl C₁-C₃.

In another preferred embodiment, X is S. More preferably R₁ is H, X is S. Even more preferably, R₁ is H, X is S and R₂, R₃ and R₄ are independently selected from H, NO₂ and NH₂.

In another preferred embodiment, X is CO. More preferably, X is CO and R₁ is H. Even more preferably, X is CO, R₁ is H and R₂, R₃ and R₄ are independently selected from halogen and H.

In another embodiment of the present invention for use, R₁ is NH₂. More preferably, R₁ is NH₂ and X is O. Even more preferably, R₁ is NH₂, X is O and R₂, R₃ and R₄ are independently selected from: H, halogen and CF₃, at least one of them being different from H.Even more preferably, R₁ is NH₂, X is O, R₄ is H and R₂ and R₃ are independently selected from: H, halogen and CF₃, at least one of them being different from H.

In a more preferred embodiment of this first aspect, the invention relates to the compounds of formula (I) for use selected from the list comprising: *N*-(4-phenoxyphenyl)-*7H*-pyrrolo[2,3-*d*]pyrimidin-4-amine *N*-[4-(4-chlorophenoxy)phenyl]-7*H*-pyrrolo[2,3-*d*]pyrimidin-4-amine *N*-[4-(4-fluorophenoxy)phenyl]-7*H*-pyrrolo[2,3-*d*]pyrimidin-4-amine *N*-[4-(4-cyanophenoxy)phenyl]-7H-pyrrolo[2,3-*d*]pyrimidin-4-amine *N*-[4-(4-methoxyphenoxy)phenyl]-*7H*-pyrrolo[2,3-*d*]pyrimidin-4-amine *N*-[4-(4-trifluoromethylphenoxy)phenyl]-*7H*-pyrrolo[2,3-*d*]pyrimidin-4-amine *N*-[4-(4-bromophenoxy)phenyl]-*7H*-pyrrolo[2,3-*d*]pyrimidin-4-amine *N*-[4-(4-nitrophenoxy)phenyl]-*7H*-pyrrolo[2,3-*d*]pyrimidin-4-amine *N*-[4-(4-aminophenoxy)phenyl]-7*H*-pyrrolo[2,3-*d*]pyrimidin-4-amine *N*-[4-(4-methylphenoxy)phenyl]-*7H*-pyrrolo[2,3-*d*]pyrimidin-4-amine *N*-[4-(3-methylphenoxy)phenyl]-*7H*-pyrrolo[2,3-*d*]pyrimidin-4-amine *N*-[4-(3-chlorophenoxy)phenyl]-*7H*-pyrrolo[2,3-*d*]pyrimidin-4-amine *N*-[4-(3-trifluoromethylphenoxy)phenyl]-*7H*-pyrrolo[2,3-*d*]pyrimidin-4-amine *N*-[4-(2-chlorophenoxy)phenyl]-*7H*-pyrrolo[2,3-*d*]pyrimidin-4-amine *N*-[4-(2-methoxyphenoxy)phenyl]-*7H*-pyrrolo[2,3-*d*]pyrimidin-4-amine *N*-[4-(2.4-chlorophenoxy)phenyl]-*7H*-pyrrolo[2,3-*d*]pyrimidin-4-amine *N*-[4-(3-methoxyphenoxy)phenyl]-*7H*-pyrrolo[2,3-*d*]pyrimidin-4-amine *N*4-(4-(4-(trifluoromethyl)phenoxy)phenyl]-*7H*-pyrrolo[2,3-*d*]pyrimidine-2,4-diamine *N*4-(4-(3-(trifluoromethyl)phenoxy)phenyl]-*7H*-pyrrolo[2,3-*d*]pyrimidine-2,4-diamine *N*4-(4-(4-chlorophenoxy)phenyl]-*7H*-pyrrolo[2,3-*d*]pyrimidine-2,4-diamine *N*4-(4-(3-chlorophenoxy)phenyl)-*7H*-pyrrolo[2,3-*d*]pyrimidine-2,4-diamine *N*-[4-((4-nitrophenyl)thio)phenyl]-*7H*-pyrrolo[2,3-*d*]pyrimidin-4-amine *N*-[4-((4-aminophenyl)thio)phenyl]-*7H*-pyrrolo[2,3-*d*]pyrimidin-4-amine *N*-[4-(benzyloxy)phenyl]-*7H*-pyrrolo[2,3-*d*]pyrimidin-4-amine (4-((*7H*-pyrrolo[2,3-*d*]pyrimidin-4-yl)amino)phenyl](phenyl)methanone (4-((*7H*-pyrrolo[2,3-*d*]pyrimidin-4-yl)amino)phenyl](4-fluorophenyl)methanone.

The structure of each of the compounds mentioned in the above list is indicated in the examples (See example 1).

A second aspect of the invention relates to a compound of formula (I) 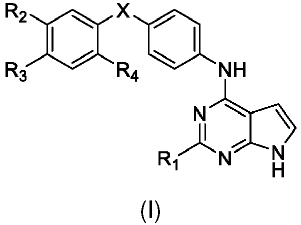 or any of the isomers thereof or the pharmaceutically acceptable salts thereof, wherein R₁, X, R₂, R₃ and R₄ have the same meaning described above in the first aspect of the invention and wherein at least one of R₂, R₃ and R₄ are H, provided that the compound is neither of the following two (I') and (I"):

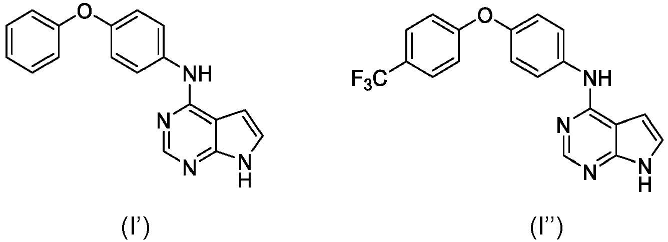

In a preferred embodiment, the invention relates to the compounds of formula (I) selected from the list comprising: *N*-[4-(4-chlorophenoxy)phenyl]-*7H*-pyrrolo[2,3-*d*]pyrimidin-4-amine *N*-[4-(4-fluorophenoxy)phenyl]-7*H*-pyrrolo[2,3-*d*]pyrimidin-4-amine *N*-[4-(4-cyanophenoxy)phenyl]-7H-pyrrolo[2,3-*d*]pyrimidin-4-amine *N*-[4-(4-methoxyphenoxy)phenyl]-*7H*-pyrrolo[2,3-*d*]pyrimidin-4-amine *N*-[4-(4-bromophenoxy)phenyl]-*7H*-pyrrolo[2,3-*d*]pyrimidin-4-amine *N*-[4-(4-nitrophenoxy)phenyl]-*7H*-pyrrolo[2,3-*d*]pyrimidin-4-amine *N*-[4-(4-aminophenoxy)phenyl]-*7H*-pyrrolo[2,3-*d*]pyrimidin-4-amine *N*-[4-(4-methylphenoxy)phenyl]-*7H*-pyrrolo[2,3-*d*]pyrimidin-4-amine *N*-[4-(3-methylphenoxy)phenyl]-*7H*-pyrrolo[2,3-*d*]pyrimidin-4-amine *N*-[4-(3-chlorophenoxy)phenyl]-*7H*-pyrrolo[2,3-*d*]pyrimidin-4-amine *N*-[4-(3-trifluoromethylphenoxy)phenyl]-*7H*-pyrrolo[2,3-*d*]pyrimidin-4-amine *N*-[4-(2-chlorophenoxy)phenyl]-*7H*-pyrrolo[2,3-*d*]pyrimidin-4-amine *N*-[4-(2-methoxyphenoxy)phenyl]-*7H*-pyrrolo[2,3-*d*]pyrimidin-4-amine *N*-[4-(2.4-chlorophenoxy)phenyl]-*7H*-pyrrolo[2,3-*d*]pyrimidin-4-amine *N*-[4-(3-methoxyphenoxy)phenyl]-*7H*-pyrrolo[2,3-*d*]pyrimidin-4-amine *N*4-(4-(4-(trifluoromethyl)phenoxy)phenyl]-*7H*-pyrrolo[2,3-*d*]pyrimidine-2,4-diamine *N*4-(4-(3-(trifluoromethyl)phenoxy)phenyl]-*7H*-pyrrolo[2,3-*d*]pyrimidine-2,4-diamine *N*4-(4-(4-chlorophenoxy)phenyl]-*7H*-pyrrolo[2,3-*d*]pyrimidine-2,4-diamine *N*4-(4-(3-chlorophenoxy)phenyl)-*7H*-pyrrolo[2,3-*d*]pyrimidine-2,4-diamine *N*-[4-((4-nitrophenyl)thio)phenyl]-*7H*-pyrrolo[2,3-*d*]pyrimidin-4-amine *N*-[4-((4-aminophenyl)thio)phenyl]-*7H*-pyrrolo[2,3-*d*]pyrimidin-4-amine *N*-[4-(benzyloxy)phenyl]-*7H*-pyrrolo[2,3-*d*]pyrimidin-4-amine *N*(4-((*7H*-pyrrolo[2,3-*d*]pyrimidin-4-yl)amino)phenyl](phenyl)methanone *N*(4-((*7H*-pyrrolo[2,3-*d*]pyrimidin-4-yl)amino)phenyl](4-fluorophenyl)methanone

A third aspect of the invention relates to a compound of formula (I) or to any of the isomers thereof or the pharmaceutically acceptable salts thereof as defined in the first aspect of the invention provided that the compound is neither (I') nor (I"), the formula of which has been indicated above, for use thereof as a medicinal product.

The invention also relates to a pharmaceutical composition comprising a compound of formula (I) or any of the isomers thereof or the pharmaceutically acceptable salts thereof as defined in the first aspect of the invention provided that the compound is neither (I') nor (I"), the formula of which has been indicated above.

The term "pharmaceutical composition" refers to the composition that includes the compound of formula (I), except for (I') and (I"), the isomers thereof or the pharmaceutically acceptable salts thereof, and at least one excipient, pharmaceutically acceptable adjuvant and/or carrier.

The term "excipients, adjuvants and/or carriers" refers to molecular entities or substances through which the active ingredient is administered. Said pharmaceutical excipients, adjuvants or carriers can be sterile liquids, like water and oils, including those of oil, animal, plant or synthetic origin, such as peanut oil, soybean oil, mineral oil, sesame oil and similar oils, excipients, disintegrating agents, wetting agents or diluents. Pharmaceutically acceptable excipients and carriers are described in "Remington's Pharmaceutical Sciences" by E.W. Martin.

The compounds of the present invention of formula (I) for use have the ability to cross the blood-brain barrier, as shown below in some of the examples. This represents an additional advantage of the compounds when using them in therapeutic treatments of diseases related to the central nervous system, such as tauopathies and TDP-43 diseases.

The compounds of the invention represented by formula (I) may be in crystalline form as free compounds or as solvates and both forms are intended to be within the scope of the present invention. The nature of the pharmaceutically acceptable solvate is not critical as long as it is pharmaceutically acceptable. In a particular embodiment, the solvate is a hydrate. The solvates can be obtained by conventional solvation methods known to those skilled in the art.

The compounds of formula (I) for therapeutic use are prepared in solid form or aqueous suspension, in a pharmaceutically acceptable diluent. These preparations can be administered by any appropriate route of administration, for which said preparation will be formulated in the pharmaceutical form suitable for the chosen route of administration. In a particular embodiment, the administration of the compound of formula (I) is carried out orally, topically, rectally or parenterally (including subcutaneously, intraperitoneally, intradermally, intramuscularly, intravenously, etc.).

Another aspect of the invention relates to a method for treating tauopathies and/or TDP-43 diseases comprising administering a therapeutically effective amount of a compound of formula (I) as described in the first aspect of the present invention to a subject.

A final aspect of the present invention refers to the use of a compound of formula (I) as described in the first aspect of the present invention for the preparation of a medicinal product for the treatment and/or prevention of tauopathies and/or TDP-43 diseases.

The compounds described in the present invention, the pharmaceutically acceptable salts thereof, solvates, as well as the pharmaceutical compositions containing them can be used together with other additional drugs in order to provide a combination therapy. Said additional drugs can make up part of the same pharmaceutical composition or, alternatively, they can be provided in the form of a separate composition for the administration thereof which is simultaneous or not to that of the pharmaceutical composition comprising a compound of formula (I), an isomer or a pharmaceutically acceptable salt thereof.

Unless indicated otherwise, the compounds of the invention also include compounds that differ only in the presence of one or more isotopically enriched atoms. For example, compounds that have said structure, with the exception of the substitution of hydrogen for deuterium or tritium, or the substitution of a carbon for a ¹³C or ¹⁴C enriched carbon or a ¹⁵N enriched nitrogen, are within the scope of this invention.

In the present invention, the term "C₁-C₃ alkyl" refers, in the present invention, to linear or branched hydrocarbon chain radicals, that have 1 to 3 carbon atoms and are joined to the rest of the molecule by a single bond, for example, propyl, ethyl, methyl, isopropyl, etc. When this substituent is present in the compounds of the invention, the "C₁-C₃ alkyl" is preferably methyl.

The term "halogen" refers to F, Cl, Br and I. When this substituent is present in the compounds of the invention, the halogen is preferably Cl.

The expression "treatment and/or prevention" as used herein, unless indicated otherwise, means to revert, alleviate, inhibit the progress of, or prevent the disorder or condition to which it applies in such terms, one or more symptoms of such disorder or condition.

Throughout the description and the claims, the word "comprises" and its variants are not intended to exclude other technical features, additives, components or steps. For those skilled in the art, other objects, advantages and features of the invention may be partially deduced from both the description and the embodiment of the invention. The following examples are provided by way of illustration and are not intended to limit the present invention.

### DESCRIPTION OF THE FIGURES

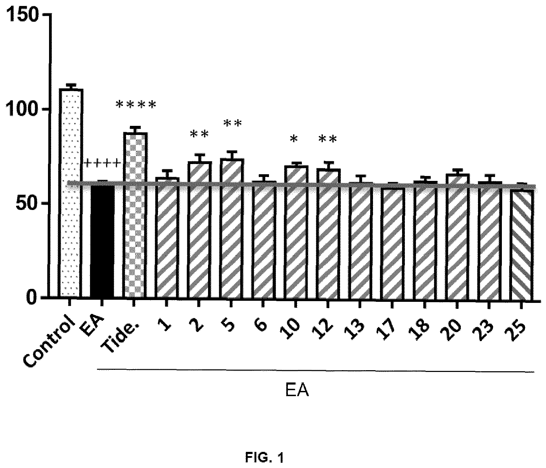

**Fig. 1****:** Cell viability (MTT) in the SH-SY5Y cell line, 24 hours after being exposed to 40 µM of ethacrynic acid (EA) in the presence or absence of the compounds of the patent (5 µM). The commercial GSK-3β inhibitor Tideglusib (5 µM) was used as an internal control. Values represent the mean ± SEM of 3 different experiments. (**p* <0.05; ***p* <0.01, *****p* <0.0001 with significant differences compared to cells treated with EA and ++++*p* <0.0001 with respect to the control).

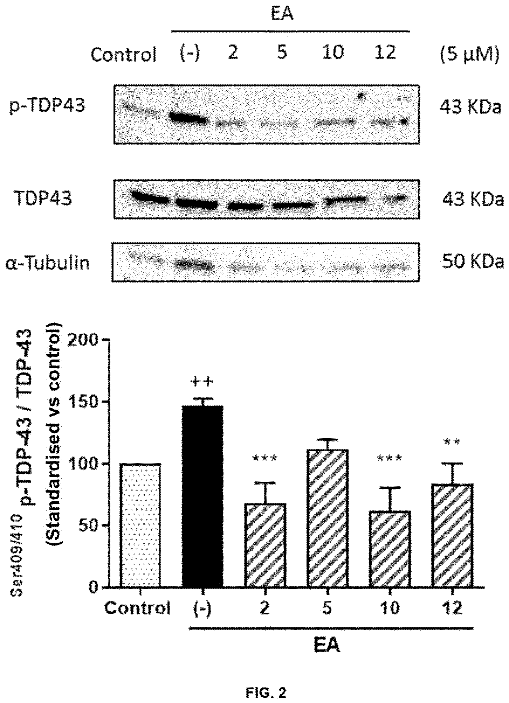

**Fig. 2****:** Representative immunoblot and quantification of p-TDP-43 levels in the SH-SY5Y line, 24 hours after being exposed to 40 µM of ethacrynic acid (EA) in the presence or absence of the compounds of the patent (5 µM). Values represent the mean ± SEM of 3 different experiments. (*p <0.05, **p <0.01 with significant differences compared to cells treated with EA and ++p <0.01 with respect to the control).

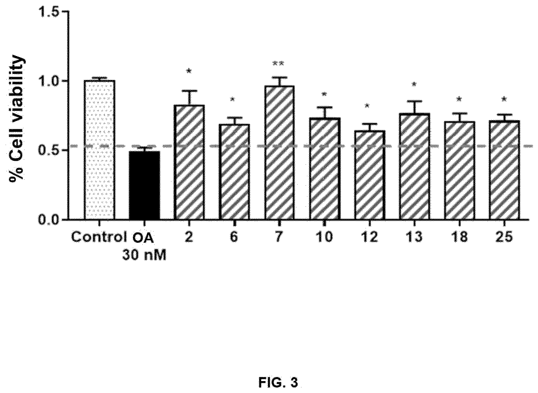

**Fig. 3****:** Cell viability (MTT) in the SH-SY5Y cell line, 24 hours after being exposed to 30 nM of okadaic acid (OA) in the presence or absence of the compounds of the patent (1 µM). Values represent the mean ± SEM of 3 different experiments. (**p* <0.05; ***p* <0.01, *****p* <0.0001 with significant differences compared to cells treated with AO and ⁺⁺⁺⁺*p* <0.0001 with respect to the control).

### EXAMPLES

Next, the invention will be illustrated by means of assays carried out by the inventors which demonstrate the effectiveness of the product of the invention.

### Example 1: Synthesis of the compounds of the invention

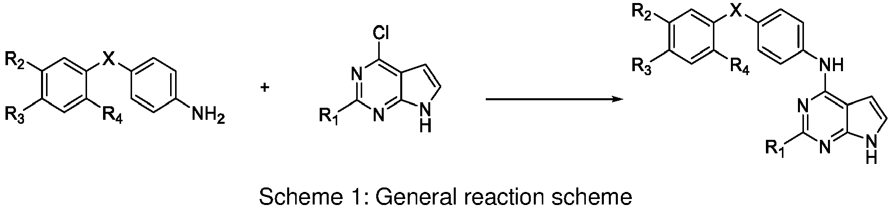

**General methodology:** 1 equivalent (250 mg, 1.63 mmol) of 4-chloro-*7H*-pyrrolo[2,3-*d*]pyrimidin-2-amine or 4-chloro-*7H*-pyrrolo[2,3-*d*]pyrimidine and 1 equivalent (1.63 mmol) of the corresponding aniline (both reagents commercially available from SigmaAldrich or Fluorochem) are dissolved in 2 ml of tetrahydrofuran. The crude mixture is stirred under microwave radiation at 100 °C until the reaction is complete. Then, the crude mixture is extracted with 20 ml of ethyl acetate and washed with saturated NaHCOs and NaCl solutions, the organic phases are combined and dried with MgSO₄, they are filtered and the solvent is removed under reduced pressure. The resulting crude mixture is purified using column chromatography using an appropriate mixture of DCM and MeOH as eluents.

### (1) N-{4-phenoxyphenyl)-7H-pyrrolo[2,3-d]pyrimidin-4-amine

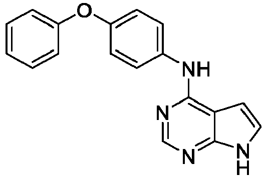

Reagents: 4-chloro-*7H*-pyrrolo[2,3-*d*]pyrimidine and 4-phenoxyaniline
m.p.: 250-251 °C. ¹H-NMR (300 MHz, DMSO-*d*₆): δ (ppm) 11.75 (s, 1H), 9.34 (s, 1H), 8.26 (s, 1H), 7.89 (d, *J* = 9.0 Hz, 2H), 7.37 (dd, *J* = 8.6, 7.3 Hz, 2H), 7.23 (dd, *J* = 3.5, 2.3 Hz, 1H), 7.13 - 6.96 (m, 5H), 6.77 (dd, *J* = 3.5, 1.9 Hz, 1H). ¹³C-NMR (75 MHz, DMSO-*d*₆) δ(ppm) 157.6, 153.5, 150.8, 150.75, 150.7, 136.3, 129.9, 122.7, 122.1, 122.0, 119.4, 117.6, 103.5, 98.7. Elemental analysis C₁₈H₁₄N₄O Calculated %C 71.51, %H 7.67, %N 18.53 Found %C 71.04, %H 4.70, %N 18.38.

### (2) N-[4-(4-chlorophenoxy)phenyl]-7H-pyrrolo[2,3-d]pyrimidin-4-amine

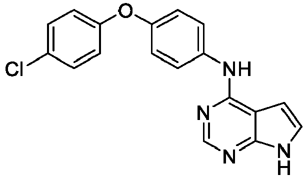

Reagants:4-chloro-*7H*-pyrrolo[2,3-*d*]pyrimidine and 4-(4-chlorophenoxy)aniline m.p.: 250-251 °C. ¹H-NMR (300 MHz, DMSO-*d*₆): δ (ppm) 11.77 (s, 1H), 9.36 (s, 1H), 8.26 (s, 1H), 7.92 (d, *J* = 9.0 Hz, 2H), 7.41 (d, *J* =9.0 Hz, 2H), 7.24 (dd, *J* = 3.4, 2.3 Hz, 1H), 7.06 (d, *J* = 9.0 Hz, 2H), 7.00 (d, *J* =9.0 Hz, 2H), 6.77 (dd, *J* = 3.5, 1.9 Hz, 1H). ¹³C-NMR (75 MHz, DMSO-*d*₆) δ 156.7, 153.4, 150.8, 150.7, 150.2, 136.8, 129.7, 126.4, 122.1, 121.9, 119.7, 119.2, 103.5, 98.7. C₁₈H₁₃ClN₄O Theoretical %C 64.20, %H 3.89, %N 16.64 Experimental %C 65.15, %H 3.96, %N 16.69.

### (3) N-[4-(4-fluorophenoxy)phenyl]-7H-pyrrolo[2,3-d]pyrimidin-4-amine

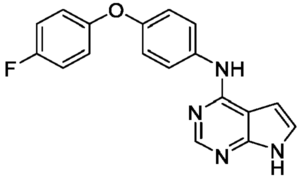

Reagents: 4-chloro-*7H*-pyrrolo[2,3-*d*]pyrimidine and 4-(4-fluorophenoxy)aniline m.p.: 227-228 °C. ¹H-NMR (300 MHz, DMSO-*d*₆): δ (ppm) 11.75 (s, 1H), 9.33 (s, 1H), 8.25 (s, 1H), 7.88 (d, *J* = 9.0 Hz, 2H), 7.26-7.15 (m, 3H), 7.08-6.95 (m, 4H), 6.76 (dd, *J* = 3.5, 1.9 Hz, 1H). ¹³C-NMR (75 MHz, DMSO-*d*₆): δ (ppm) 159.4 (d, *J* = 238.1 Hz, 153.6 (d, *J* = 2.3 Hz), 153.5, 151.3, 150.8, 136.3, 122.1, 122.0, 119.6 (d, *J* =8.4 Hz), 119.0, 116.4 (d, *J* = 23.3 Hz), 103.5, 98.7. C₁₈H₁₃FN₄O Theoretical %C 67.49, %H 4.09, %N 17.49 Experimental %C 67.09, %H 4.09, %N 17.38.

### (4) N-[4-(4-cyanophenoxy)phenyl]-7H-pyrrolo[2,3-d]pyrimidin-4-amine

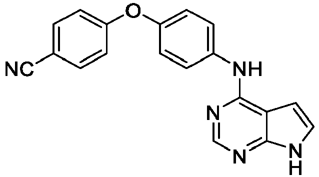

Reagents: 4-chloro-*7H*-pyrrolo[2,3-*d*]pyrimidine and 4-(4-cyanophenoxy)aniline m.p.: 259-260 °C. ¹H-NMR (300 MHz, DMSO-*d*₆): δ (ppm) 11.77 (s, 1H), 9.41 (s, 1H), 8.28 (s, 1H), 7.99 (d, *J* = 9.0 Hz, 2H), 7.83 (d, *J* = 8.9 Hz, 2H), 7.25 (dd, *J* = 3.5, 2.3 Hz, 1H), 7.15 (d, *J* = 9.0 Hz, 2H), 7.09 (d, *J* =8.9 Hz, 2H), 6.79 (dd, *J* =3.5, 1.8 Hz, 1H). ¹³C-NMR (75 MHz, DMSO-*d*₆): δ (ppm) 161.9, 153.3, 150.8, 150.7, 148.4, 137.8, 134.6, 122.2, 121.8, 120.7, 118.8, 117.3, 104.5, 103.6, 98.7. C₁₉H₁₃N₅O Theoretical %C 69.71, %H 4.00, %N 21.39 Experimental %C 69.33, %H 4.15, %N 21.01.

### (5) N-[4-(4-methoxyphenoxy)phenyl]-7H-pyrrolo[2,3-d]pyrimidin-4-amine

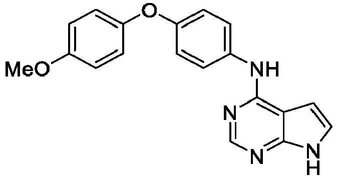

Reagents: 4-chloro-*7H*-pyrrolo[2,3-*d*]pyrimidine and 4-(4-methoxyphenoxy)aniline. m.p.: 202-203 °C. ¹H-NMR (300 MHz, DMSO-*d*₆): δ (ppm) 11.74 (s, 1H), 9.30 (s, 1H), 8.25 (s, 1H), 7.84 (d, *J* = 9.0 Hz, 2H), 7.23 (dd, *J* = 3.5, 2.3 Hz, 1H), 7.04-6.94 (m, 6H), 6.76 (dd, *J* = 3.1, 1.9 Hz, 1H), 3.76 (s, 3H). ¹³C-NMR (75 MHz, DMSO-*d*₆): δ (ppm) 155.2, 153.5, 152.4, 150.8, 150.7, 150.4, 135.5, 122.0, 121.9, 119.8, 118.0, 115.0, 103.4, 98.7, 55.4. C₁₉H₁₆N₄O₂ Theoretical %C 68.66, %H 4.85, %N 16.86 Experimental %C 67.97, %H 4.91, %N 16.64.

### (6) [N-[4-(4-trifluoromethylphenoxy)phenyl]-7H-pyrrolo[2,3-d]pyrimidin-4-amine

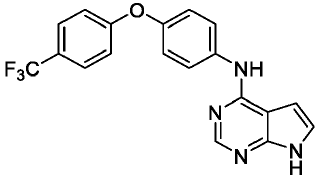

Reagents: 4-chloro-*7H*-pyrrolo[2,3-*d*]pyrimidine and 4-(4-trifluoromethylphenoxy)aniline m.p.: 233-234 °C. ¹H-NMR (300 MHz, DMSO-*d*₆): δ (ppm) 11.77 (s, 1H), 9.40 (s, 1H), 8.28 (s, 1H), 7.98 (d, *J* = 9.0 Hz, 2H), 7.72 (d, *J* = 8.7 Hz, 2H), 7.24 (dd, *J* = 3.5, 2.3 Hz, 1H), 7.14 (t, *J* = 9.1 Hz, 4H), 6.79 (dd, *J* = 3.5, 1.9 Hz, 1H).¹³C-MNR (75 MHz, DMSO-*d*₆): δ (ppm) 162.1, 154.3, 151.7, 151.6, 149.9, 138.4, 128.3 (q, *J* = 3.6 Hz), 125.2 (q, *J* = 271.7 Hz), 123.6(q, *J* = 32.1 Hz), 123.1, 122.7, 121.4, 118.0, 104.5, 99.6. C₁₉H₁₃F₃N₄O Theoretical %C 61.62, %H 3.54, %N 15.13 Experimental %C 61.55, %H 3.51, %N 15.04.

### (7) [N-[4-(4-bromophenoxy)phenyl]-7H-pyrrolo[2,3-d]pyrimidin-4-amine

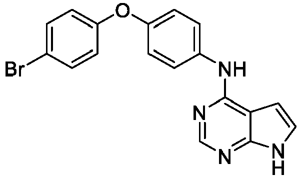

Reagents: 4-chloro-*7H*-pyrrolo[2,3-*d*]pyrimidine and 4-(4-bromophenoxy)aniline m.p.: 257-258 °C. ¹H-NMR (300 MHz, DMSO-*d*₆): δ (ppm) 11.75 (s, 1H), 9.35 (s, 1H), 8.27 (s, 1H), 7.93 (d, *J* = 9.0 Hz, 2H), 7.54 (d, *J* = 9.0 Hz, 2H), 7.24 (dd, *J* = 3.5, 2.3 Hz, 1H), 7.08 (d, *J* = 9.0 Hz, 2H), 6.96 (d, *J* = 8.9 Hz, 2H), 6.78 (dd, *J* = 3.5, 1.9 Hz, 1H). ¹³C-NMR (75 MHz, DMSO-*d*₆) δ 157.7, 153.9, 151.3, 151.2, 150.6, 137.3, 133.1, 122.6, 122.4, 120.2, 120.1, 114.7, 104.0, 99.2. C₁₈H₁₃BrN₄O Theoretical %C 56.71, %H 3.44, %N 14.70 Experimental %C 56.21, %H 3.51, %N 14.43.

### (8) [N-[4-(4-nitrophenoxy)phenyl]-7H-pyrrolo[2,3-d]pyrimidin-4-amine

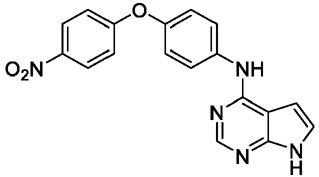

Reagents: 4-chloro-*7H*-pyrrolo[2,3-*d*]pyrimidine and 4-(4-nitrophenoxy)aniline m.p.: 270 °C. ¹H-NMR (300 MHz, DMSO-*d*₆): δ (ppm) 11.86 (s, 1H), 9.63 (s, 1H), 8.35 - 8.16 (m, 3H), 8.00 (d, *J* = 9.0 Hz, 2H), 7.29 - 7.24 (m, 1H), 7.20 (d, *J* = 9.0 Hz, 2H), 7.14 (d, *J* = 9.3 Hz, 2H), 6.84 (dd, *J* = 3.5, 1.9 Hz, 1H). ¹³C-NMR (75 MHz, DMSO-*d*₆) δ 163.6, 153.1, 150.5, 150.1, 148.7, 142.0, 137.7, 126.2, 122.5, 122.3, 120.9, 116.9, 103.6, 99.1. ESI calculated for C₁₈H₁₃N₅O₃ [M + H]⁺ 348.1091 found 348.1088.

### (9) [N-[4-(4-aminophenoxy)phenyl]-7H-pyrrolo[2,3-d]pyrimidin-4-amine

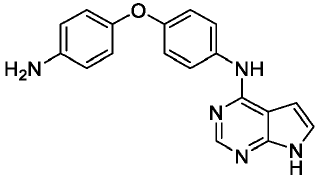

Reagents: 4-chloro-*7H*-pyrrolo[2,3-*d*]pyrimidine and 4-(4-aminophenoxy)aniline m.p.: 242-243 °C. ¹H-NMR (300 MHz, DMSO-*d*₆): δ (ppm) 11.68 (s, 1H), 9.21 (s, 1H), 8.21 (s, 1H), 7.75 (d, *J* = 9.0 Hz, 2H), 7.19 (dd, J= 3.5, 2.3 Hz, 1H), 6.87 (d, *J* = 9.0 Hz, 2H), 6.76 (d, *J* = 8.8 Hz, 2H), 6.71 (dd, *J* = 3.5, 1.9 Hz, 1H), 6.58 (d, *J* = 8.8 Hz, 2H), 4.92 (s, 2H). ¹³C-NMR (75 MHz, DMSO-*d*₆) δ 153.74, 153.64, 150.8, 150.7, 146.5, 145.1, 134.7, 122.1, 121.9, 120.3, 117.0, 114.8, 103.3, 98.7. ESI calculated for C₁₈H₁₅N₅O [M + H]⁺ 318.1349 found 318.1348.

### (10) [N-[4-(4-methylphenoxy)phenyl]-7H-pyrrolo[2,3-d]pyrimidin-4-amine

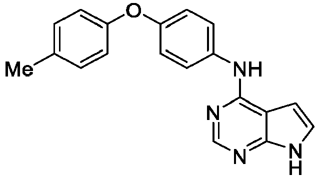

Reagents: 4-chloro-*7H*-pyrrolo[2,3-*d*]pyrimidine and 4-(4-methylphenoxy)aniline m.p.: 229 °C. ¹H-NMR (300 MHz, DMSO-*d*₆): δ (ppm) 11.72 (s, 1H), 9.29 (s, 1H), 8.24 (s, 1H), 7.85 (d, *J* = 9.0 Hz, 2H), 7.22 (dd, *J* = 3.5, 2.4 Hz, 1H), 7.17 (dd, *J* = 8.8, 0.7 Hz, 2H), 6.99 (d, *J* = 9 Hz, 2H), 6.89 (d, *J* = 8.5 Hz, 2H), 6.75 (dd, *J* = 3.5, 1.9 Hz, 1H), 2.28 (s, 3H). ¹³C-NMR (75 MHz, DMSO-*d*₆): δ (ppm) 155.2, 153.5, 151.5, 150.8 (C-2, 4), 136.0, 131.9, 130.3, 122.03, 121.98, 118.9, 117.9, 103.5, 98.7, 20.2. C₁₉H₁₆N₄O Theoretical %C 72.13, %H 5.10, %N 17.71 Experimental %C 71.84, %H 5.15, %N 17.96.

### (11) [N-[4-(3-methylphenoxy)phenyl]-7H-pyrrolo[2,3-d]pyrimidin-4-amine

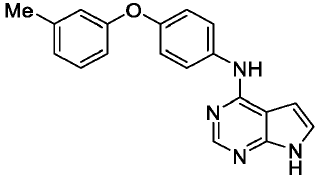

Reagents: 4-chloro-*7H*-pyrrolo[2,3-*d*]pyrimidine and 4-(3-methylphenoxy)aniline m.p.: 212-213 °C. ¹H-NMR (300 MHz, DMSO-*d*₆): δ (ppm) 11.73 (s, 1H), 9.31 (s, 1H), 8.25 (s, 1H), 7.88 (d, *J* = 9.0 Hz, 2H), 7.28-7.20 (m, 2H), 7.02 (d, *J* = 9.0 Hz, 2H), 6.91 (ddt, *J* = 7.5, 1.7, 0.8 Hz, 1H), 6.82-6.78 (m, 3H), 2.28 (s, 3H). ¹³C-NMR (75 MHz, DMSO-*d*₆) δ (ppm) 157.7, 153.5, 150.9, 150.79, 150.76, 139.6, 136.3, 129.6, 123.5, 122.1, 121.9, 119.4, 118.1, 114.7, 103.5, 98.7, 21.0. ESI calculated for C₁₉H₁₆N₄O [M + H]⁺ 317.1397 found 317.1393.

### (12) [N-[4-(3-chlorophenoxy)phenyl]-7H-pyrrolo[2,3-d]pyrimidin-4-amine

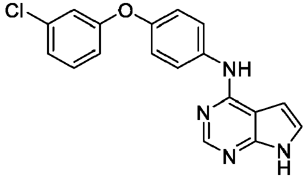

Reagents: 4-chloro-*7H*-pyrrolo[2,3-*d*]pyrimidine and 4-(3-chlorophenoxy)aniline m.p.: 224-225 °C. ¹H-NMR (300 MHz, DMSO-*d*₆): δ (ppm) 11.79 (s, 1H), 9.39 (s, 1H), 8.29 (s, 1H), 7.96 (d, *J* = 9.0 Hz, 2H), 7.41 (t, *J* = 8.1 Hz, 1H), 7.26 (dd, *J* = 3.5, 2.2 Hz, 1H), 7.17 (ddd, *J* = 8.0, 2.0, 0.9 Hz, 1H), 7.12 (d, *J* = 9.0 Hz, 2H), 7.03 (t, *J* = 2.2 Hz, 1H), 6.97 (ddd, *J* = 8.3, 2.3, 0.8 Hz, 1H), 6.80 (dd, *J* = 3.5, 1.8 Hz, 1H). ¹³C-NMR (75 MHz, DMSO-*d*₆): δ (ppm) 158.9, 153.4, 150.8, 150.8, 149.7, 137.1, 133.9, 131.4, 122.5, 122.2, 121.9, 120.0, 117.2, 116.0, 103.5, 98.7. C₁₈H₁₃ClN₄O Theoretical %C 64.20, %H 3.89, %N 16.64 Experimental %C 64.06, %H 3.91, %N 16.75.

### (13) [N-[4-(3-trifluoromethylphenoxy)phenyl]-7H-pyrrolo[2,3-d]pyrimidin-4-amine

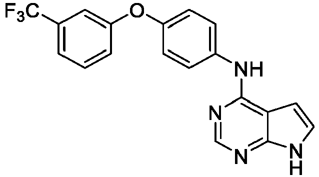

Reagents: 4-chloro-*7H*-pyrrolo[2,3-*d*]pyrimidine and 4-(3-trifluoromethylphenoxy)aniline m.p.: 176-177 °C. ¹H-NMR (300 MHz, DMSO-*d*₆): δ (ppm) 11.75 (s, 1H), 9.37 (s, 1H), 8.27 (s, 1H), 7.96 (d, *J* = 9.0 Hz, 2H), 7.61 (t, *J* = 7.8 Hz, 1H), 7.44 (d, *J* = 7.9 Hz, 1H), 7.32 - 7.19 (m, 3H), 7.13 (d, *J* = 8.9 Hz, 2H), 6.78 (dd, J= 3.5, 1.9 Hz, 1H). ¹³C-NMR (75 MHz, DMSO-*d*₆): δ (ppm) 158.8, 153.8, 151.2, 151.1, 149.9, 137.6, 131.6, 130.95 (q, *J =* 32 Hz), 127.7(d, *J* = 272.5 Hz), 122.5, 122.3, 121.5, 120.5, 119.5 (d, *J* = 3.9 Hz), 113.9 (d, *J* = 4.0 Hz), 103.9, 99.1. C₁₉H₁₃F₃N₄O Theoretical %C 61.62, %H 3.54, %N 15.13 Experimental %C 61.60, %H 3.47, %N 15.22.

### (14) [N-[4-(2-chlorophenoxy)phenyl]-7H-pyrrolo[2,3-d]pyrimidin-4-amine

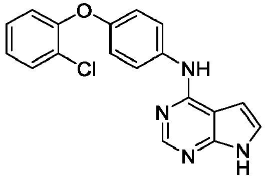

Reagents: 4-chloro-*7H*-pyrrolo[2,3-*d*]pyrimidine and 4-(2-chlorophenoxy)aniline m.p.: 215 °C. ¹H-NMR (300 MHz, DMSO-*d*₆): δ (ppm) 11.73 (s, 1H), 9.33 (s, 1H), 8.25 (s, 1H), 7.89 (d, *J* = 9.0 Hz, 2H), 7.58 (dd, *J* = 8.0, 1.6 Hz, 1H), 7.34 (ddd, *J* = 8.2, 7.4, 1.6 Hz, 1H), 7.22 (dd, *J* = 3.5, 2.4 Hz, 1H), 7.17 (ddd, *J* = 8.0, 7.4, 1.5 Hz, 1H), 7.05-6.95 (m, 3H), 6.76 (dd, *J* = 3.5, 1.9 Hz, 1H). ¹³C-NMR (75 MHz, DMSO-*d*₆):δ (ppm) 153.5, 152.6, 150.80, 150.74, 150.71, 136.5, 130.6, 128.7, 124.6, 123.8, 122.1, 122.0, 119.8, 118.4, 103.5, 98.7. C₁₈H₁₃ClN₄O Theoretical %C 64.20, %H 3.89, %N 16.64 Experimental %C 64.11, %H 3.96, %N 16.63.

### (15) [N-[4-(2-methoxyphenoxy)phenyl]-7H-pyrrolo[2,3-d]pyrimidin-4-amine

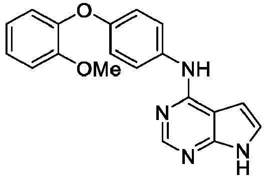

Reagents: 4-chloro-*7H*-pyrrolo[2,3-*d*]pyrimidine and 4-(2-methoxyphenoxy)aniline m.p.: 214-215 °C. ¹H-NMR (300 MHz, DMSO-*d*₆): δ (ppm) 11.69 (s, 1H), 9.23 (s, 1H), 8.21 (s, 1H), 7.76 (d, *J* = 9.0 Hz, 2H), 7.20 (dd, *J* = 3.5, 2.3 Hz, 1H), 7.18 - 7.14 (m, 2H), 7.02 - 6.92 (m, 2H), 6.86 (d, *J* = 9.1 Hz, 2H), 6.71 (dd, *J* = 3.5, 1.9 Hz, 1H), 3.77 (s, 3H). ¹³C-NMR (75 MHz, DMSO-*d*₆) δ (ppm) 153.6, 152.6, 151.1, 150.8, 150.7, 144.6, 135.0, 124.9, 122.1, 121.9, 121.0, 120.7, 116.7, 113.3, 103.3, 98.7, 55.6. C₁₉H₁₆N₄O₂ Theoretical %C 68.66, %H 4.85, %N 16.96 Experimental %C 68.006, %H 4.82, %N 16.97.

### (16) [N-[4-(2.4-chlorophenoxy)phenyl]-7H-pyrrolo[2,3-d]pyrimidin-4-amine

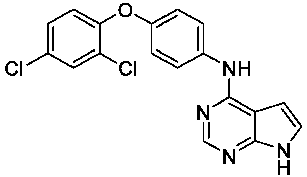

Reagents: 4-chloro-*7H*-pyrrolo[2,3-*d*]pyrimidine and 4-(2,4-dichlorophenoxy)aniline m.p.: 252-253 °C. ¹H-NMR (300 MHz, DMSO-*d*₆): δ (ppm) 11.72 (s, 1H), 9.37 (s, 1H), 8.24 (s, 1H), 7.89 (d, *J* = 9.1 Hz, 2H), 7.72 (d, *J* = 2.5 Hz, 1H), 7.39 (dd, *J* = 8.8, 2.6 Hz, 1H), 7.22 (dd, *J* = 3.5, 2.3 Hz, 1H), 7.02 (dd, *J* = 8.9, 2.1 Hz, 3H), 6.75 (dd, *J* = 3.5, 1.9 Hz, 1H). ¹³C-NMR (75 MHz, DMSO-*d*₆) δ (ppm) 153.6, 152.0, 150.9, 150.5, 136.9, 130.1, 128.8, 127.7, 124.9, 122.4, 122.2, 120.9, 118.8, 103.7, 98.9. C₁₈H₁₂Cl₂N₄O Theoretical %C 58.24, %H 3.26, %N 15.09 Experimental %C 58.13, %H 3.33, %N 15.17.

### (17) [N-[4-(3-methoxyphenoxy)phenyl]-7H-pyrrolo[2,3-d]pyrimidin-4-amine

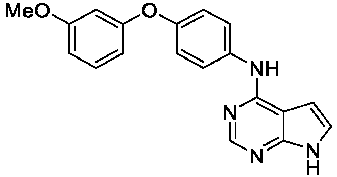

Reagents: 4-chloro-*7H*-pyrrolo[2,3-*d*]pyrimidine and 4-(3-methoxyphenoxy)aniline m.p.: 210-211 °C. 1H-NMR (300 MHz, DMSO-*d*₆): δ (ppm) 11.73 (s, 1H), 9.32 (s, 1H), 8.25 (s, 1H), 7.89 (d, *J* = 9.0 Hz, 2H), 7.31 - 7.20 (m, 2H), 7.05 (d, *J* = 9.0 Hz, 2H), 6.76 (dd, *J* = 3.5, 1.8 Hz, 1H), 6.67 (ddd, *J* = 8.3, 2.4, 0.9 Hz, 1H), 6.59 - 6.48 (m, 2H), 3.73 (s, 3H), ¹³C-NMR (75 MHz, DMSO-*d*₆):δ (ppm) 160.6, 158.9, 153.5, 150.80, 150.75, 150.5, 136.5, 130.4, 122.1, 121.9, 119.6, 109.5, 108.4, 103.7, 103.5, 98.7, 55.2, ESI calculated for C₁₉H₁₆N₄O₂ [M + H]⁺ 333.1346 found 333.1342.

### (18) N4-(4-(4-(trifluoromethyl)phenoxy)phenyl]-7H-pyrrolo[2,3-d]pyrimidine-2,4-diamine

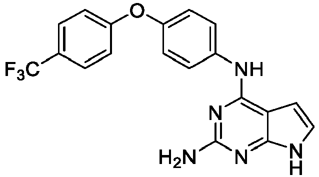

Reagents: 4-chloro-7*H*-pyrrolo[2,3-*d*]pyrimidin-2-amine and 4-(4-trifluoromethylphenoxy)aniline
m.p.: 201-202 °C. ¹H-NMR (300 MHz, DMSO-*d*₆): δ (ppm) 10.85 (s, 1H), 8.99 (s, 1H), 8.03 (d, *J* = 9.0 Hz, 2H), 7.72 (d, *J* = 9.0 Hz, 2H), 7.11 (dd, *J* = 9.0, 0.8 Hz, 2H), 7.07 (d, *J* = 9.0 Hz, 2H), 6.77 (dd, *J* = 3.5, 2.2 Hz, 1H), 6.55 (dd, *J* = 3.5, 1.9 Hz, 1H), 5.72 (s, 2H). ¹³C-NMR (75 MHz, DMSO-*d*₆) δ (ppm) 161.3, 159.4, 153.9, 153.5, 148.5, 138.2, 127.4 (q, *J* = 3.7 Hz), 124.4 (d, *J* = 271.1 Hz), 122.7 (d, *J* = 32 Hz), 121.2, 120.4, 117.9, 117.1, 108.5, 98.8, 96.9. ESI calculated for C₁₉H₁₄F₃N₅O [M + H]⁺ 386.1223 found 386.1220.

### (19) N4-(4-(3-(trifluoromethyl)phenoxy)phenyl]-7H-pyrrolo[2,3-d]pyrimidine-2,4-diamine

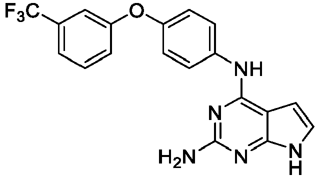

Reagents: 4-chloro-*7H*-pyrrolo[2,3-*d*]pyrimidin-2-amine and 4-(3-trifluoromethylphenoxy)aniline
m.p.: 179-180 °C. ¹H-NMR (300 MHz, DMSO-*d*₆): δ (ppm) 10.84 (s, 1H), 8.98 (s, 1H), 8.02 (d, *J* = 9.1 Hz, 2H), 7.60 (t, *J* = 7.9 Hz, 1H), 7.44 (ddt, *J* = 7.7, 1.7, 0.9 Hz, 1H), 7.32 - 7.20 (m, 2H), 7.06 (d, *J* = 9.0 Hz, 2H), 6.76 (dd, *J* = 3.5, 2.2 Hz, 1H), 6.54 (dd, *J* = 3.5, 2.0 Hz, 1H), 5.72 (s, 2H), ¹³C-NMR (75 MHz, DMSO-*d*₆) δ 159.4, 158.6, 153.9, 153.5, 148.9, 138.0, 131.3, 130.6 (d, *J* = 31.9 Hz), 123.8 (d, *J* = 272.5 Hz), 121.3, 121.1, 120.0, 119.0 (d, *J* = 4.1 Hz), 117.9, 113.4 (d, *J* = 4.0 Hz), 98.8, 96.9, ESI calculated for C₁₉H₁₄F₃N₅O [M + H]⁺ 386.1223 found 386.1219.

### (20) N4-(4-(4-chlorophenoxy)phenyl]-7H-pyrrolo[2,3-d]pyrimidine-2,4-diamine

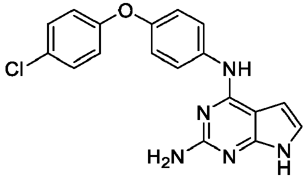

Reagents: 4-chloro-*7H*-pyrrolo[2,3-*d*]pyrimidin-2-amine and 4-(4-chlorophenoxy)aniline m.p.: 232-233 °C. ¹H-NMR (300 MHz, DMSO-*d*₆): δ (ppm) 10.83 (s, 1H), 8.94 (s, 1H), 7.98 (d, *J* = 9.1 Hz, 2H), 7.41 (d, *J* = 9.0 Hz, 2H), 6.99 (m, 4H), 6.76 (dd, *J* = 3.5, 2.1 Hz, 1H), 6.53 (dd, *J* = 3.5, 1.9 Hz, 1H), 5.70 (s, 2H). ¹³C-NMR (75 MHz, DMSO-*d*₆) δ 159.4, 156.8, 154.0, 153.5, 149.7, 137.5, 129.7, 126.3, 121.3, 119.5, 119.1, 117.8, 98.8, 96.9. ESI calculated for C₁₈H₁₄ClN₅O [M + H]⁺ 352.0960 found 352.0956.

### (21) N4-(4-(3-chlorophenoxy)phenyl)-7H-pyrrolo[2,3-d]pyrimidine-2,4-diamine

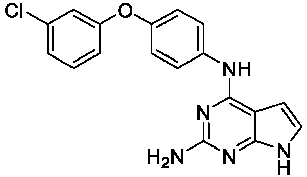

Reagents: 4-chloro-*7H*-pyrrolo[2,3-*d*]pyrimidin-2-amine and 4-(3-chlorophenoxy)aniline m.p.: 177-178 °C. ¹H-NMR (300 MHz, DMSO-*d*₆): δ (ppm) 10.84 (s, 1H), 8.97 (s, 1H), 8.00 (d, *J* = 9.0 Hz, 2H), 7.39 (t, *J* = 8.1 Hz, 1H), 7.14 (ddd, *J* = 8.0, 2.0, 0.9 Hz, 1H) 7.03 (d, *J* = 9.0 Hz, 2H) 6.99 (t, *J* = 2.2 Hz, 1H), 6.94 (ddd, *J* = 8.3, 2.4, 0.9 Hz, 1H), 6.76 (dd, *J* = 3.5, 2.2 Hz, 1H), 6.54 (dd, *J* = 3.5, 1.9 Hz, 1H), 5.71 (s, 2H). ¹³C-NMR (75 MHz, DMSO-*d*₆) δ (ppm) 159.4, 159.1, 153.9, 153.5, 149.1, 137.8, 133.9, 131.3, 122.5, 121.3, 119.9, 117.8, 117.1, 116.0, 98.8, 96.9. ESI calculated for C₁₈H₁₄ClN₅O [M + H]⁺ 352.0960 found 352.0955.

### (22) [N-[4-((4-nitrophenyl)thio)phenyl]-7H-pyrrolo[2,3-d]pyrimidin-4-amine

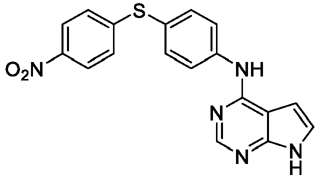

Reagents: 4-chloro-*7H*-pyrrolo[2,3-*d*]pyrimidine and 4-((4-nitrophenyl)thio)aniline m.p.: 265-266 °C ¹H-NMR (300MHz, DMSO-*d*₆): δ (ppm) 12.46 (s, 1H), 10.72 (s, 1H), 8.43 (s, 1H), 8.17 (d, *J* = 8.6 Hz, 2H), 8.00 (d, *J* = 8.1 Hz, 2H), 7.67 (d, *J* = 8.2 Hz, 2H), 7.44 (t, *J* = 2.6 Hz, 1H), 7.33 (d, *J* = 8.6 Hz, 2H), 7.03 (t, *J* = 2.4 Hz, 1H).¹³C-MNR (75 MHz, DMSO-*d*₆) δ 151.7, 148.3, 147.6, 146.6, 144.9, 139.9, 135.7, 126.4, 124.3, 124.1, 123.7, 123.5, 103.7, 100.7. C₁₈H₁₃N₅O₂S Theoretical %C 59.49, %H 3.61, %N 19.27 %S 8.82 Experimental %C 59.26, %H 3.70, %N 19.37 %S 8.95.

### (23) [N-[4-((4-aminophenyl)thio)phenyl]-7H-pyrrolo[2,3-d]pyrimidin-4-amine

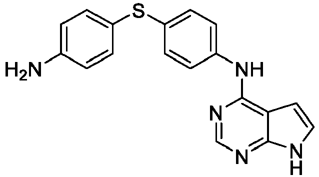

Reagents: 4-chloro-*7H*-pyrrolo[2,3-*d*]pyrimidine and 4,4'-thiodianiline m.p.: 200-201 °C. ¹H-NMR (300 MHz, DMSO-*d*₆): δ (ppm) 11.73 (s, 1H), 9.30 (s, 1H), 8.24 (s, 1H), 7.80 (d, *J* = 8.8 Hz, 2H), 7.23 (dd, *J* = 3.5, 2.3 Hz, 1H), 7.22 (dd, *J* = 3.5, 2.3 Hz, 1H), 7.14 (dd, *J* = 16.7, 8.6 Hz, 4H), 6.84 (dd, *J* = 3.5, 1.9 Hz, 1H), 6.61 (d, *J* = 8.5 Hz, 2H), 5.48 (s, 1H). ¹³C-NMR (75 MHz, DMSO-*d*₆) δ 153.3, 150.8, 150.6, 149.3, 138.5, 135.0, 131.3, 128.2, 122.1, 120.9, 117.1, 114.7, 103.6, 98.7. C₁₈H₁₅N₅S Theoretical %C 59.49, %H 3.61, %N 19.27 %S 8.82 Experimental %C 59.26, %H 3.70, %N 19.37 %S 8.95.

### (24) [N-[4-(benzyloxy)phenyl]-7H-pyrrolo[2,3-d]pyrimidin-4-amine

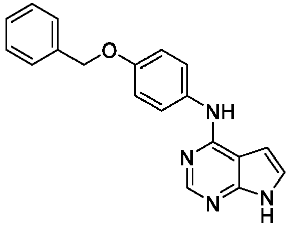

Reagents: 4-chloro-*7H*-pyrrolo[2,3-*d*]pyrimidine and 4-benzyloxyaniline
m.p.: 238-239 °C. ¹H-NMR (300 MHz, DMSO-*d*₆): δ (ppm) 11.66 (s, 1H), 9.15 (s, 1H), 8.20 (s, 1H), 7.71 (d, *J* = 9.1 Hz, 2H), 7.50-7.29 (m, 5H), 7.18 (dd, *J* = 3.5, 2.4 Hz, 1H), 7.00 (d, *J* = 9.1 Hz, 2H), 6.67 (dd, *J* = 3.5, 1.9 Hz, 1H), 5.09 (s, 2H). ¹³C-NMR (75 MHz, DMSO-*d*₆):δ (ppm) 153.80, 153.76, 150.9, 150.7, 137.3, 133.5, 128.4, 127.74, 127.66, 122.3, 122.0, 114.7, 103.2, 98.7, 69.4. ESI calculated for C₁₉H₁₆N₄O [M + H]⁺317.1397 found 317.1395.

### (25) (4-((7H-pyrrolo[2,3-d]pyrimidin-4-yl)amino)phenyl](phenyl)methanone

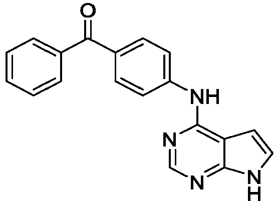

Reagents: 4-chloro-*7H*-pyrrolo[2,3-*d*]pyrimidine and (4-aminophenyl)phenylmethanone m.p.: 247-248 °C. ¹H-NMR (300 MHz, DMSO-*d*₆): δ (ppm) 11.91 (s, 1H), 9.76 (s, 1H), 8.38 (s, 1H), 8.16 (d, *J* = 8.9 Hz 2H), 7.79 (d, *J* = 8.8 Hz, 2H), 7.77-7.53 (m, 5H), 7.32 (dd, *J* = 3.5, 2.3 Hz, 1H), 6.89 (dd, *J* = 3.5, 1.9 Hz, 1H). ¹³C-NMR (75 MHz, DMSO-*d*₆): δ (ppm) 194.4, 152.8, 151.2, 150.5, 145.1, 137.9, 132.0, 131.1, 129.6, 129.3, 128.4, 123.0, 118.6, 104.4, 98.7. ESI calculated for C₁₉H₁₄N₄O [M + H]⁺ 315.1240 found 315.1238.

### (26) (4-((7H-pyrrolo[2,3-d]pyrimidin-4-yl)amino)phenyl](4-fluorophenyl)methanone

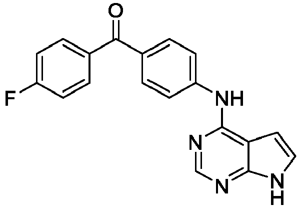

Reagents: 4-chloro-*7H*-pyrrolo[2,3-*d*]pyrimidine and (4-aminophenyl)(4'-fluorophenyl)methanone
m.p.: 275-276 °C. ¹H-NMR (300 MHz, DMSO-*d*₆): δ (ppm) 11.89 (s, 1H), 9.75 (s, 1H), 8.38 (s, 1H), 8.16 (d, *J* = 8.9 Hz, 2H), 7.93-7.72 (m, 4H), 7.39 (t, *J* = 8.9 Hz, 2H), 7.32 (dd, *J* = 3.5, 2.3 Hz, 1H), 6.88 (dd, *J* = 3.5, 2.3, 1H). ¹³C-NMR (75 MHz, DMSO-*d*₆): δ (ppm) 193.1, 164.3 (d, *J* = 250.3 Hz, 152.9, 151.2, 150.5, 145.2, 134.5 (d, *J* = 3.2 Hz), 132.2 (d, *J* = 9.2 Hz), 131.1, 129.5, 123.0, 118.7, 115.5 (d, *J* = 22.0 Hz), 104.4, 98.8.C₁₉H₁₃FN₄O Theoretical %C 68.67, %H 3.94, %N 16.86 Experimental %C 68.32, %H 3.90, %N 16.72.

### Example 2. Enzymatic inhibition of TTBK1 and TTBK2

A buffer solution containing the recombinant human enzyme TTBK1(1-1321) or TTBK2(1-450) (5-20 mU) and 50 mM Tris at pH 7.5, 0.1 mM EGTA, 0.1 % β-mercaptoethanol, 1 mg/ml BSA, 10 mM DTT, is tested against the peptide RRKDLHDDEEDEAMSITA (SEQ ID NO: 1) in a final volume of 25.5 µl. The final reaction mixture contains the peptide at a concentration of 0.3 mM, 10 mM magnesium acetate and 0.005 mM [33P-γ-ATP] (50-1000 cpm/pmol). The reaction mixture is incubated for 30 minutes at room temperature and the reaction is stopped after the addition of 5 µl of orthophosphoric acid. The mixture is placed on P81 Unifilter plates and washed with 50 mM orthophosphoric acid for data reading (see Table 1).

### Example 3. Permeability of the blood-brain barrier

The prediction of crossing the blood-brain barrier (BBB) was carried out using the PAMPA (Parallel Artificial Membrane Permeability Assay) methodology (Kansy M, Senner F, Gubernator K. Physicochemical high throughput screening: parallel artificial membrane permeation assay in the description of passive absorption processes. J Med Chem. 1998;41(7):1007-1010; Di L, Kerns EH, Fan K, McConnell OJ, Carter GT. High throughput artificial membrane permeability assay for blood-brain barrier. Eur J Med Chem. 2003;38(3):223-232). This technique consists of an artificial system of parallel plates separated by a membrane covered with porcine brain lipid that emulates the BBB. To validate the study, 10 commercial drugs approved by the FDA were used, the permeability of which in humans is known: enoxacin, hydrocortisone, desipramine, caffeine, ofloxacin, piroxicam, testosterone, promazine, verapamil and atenolol (Di L, Kerns EH, Fan K, McConnell OJ, Carter GT. High throughput artificial membrane permeability assay for blood-brain barrier. Eur J Med Chem. 2003;38(3):223-232). The study compounds and controls (1-2 mg) were dissolved in 5 ml of the buffer of the assay: phosphate buffered saline (PBS, pH 7.4) and ethanol (EtOH) in a 70:30 ratio. Next, they were filtered and subsequently scanned with the Varioskan^{™} ultraviolet (UV) reader (Thermo Fisher) from wavelength 220 to 400 nm, obtaining as a result a spectrum for each compound in which the wavelengths at which each one of them absorbs were identified.

Once the initial concentration of each compound has been determined by the absorbance obtained at the established wavelengths (initial absorbance), 180 µl of each sample was added to the 96-well donor plate, the bottom of which is a semipermeable membrane that was previously covered with 5 µl of porcine brain lipid dissolved in dodecane (20 mg/ml). The 96-well acceptor plate was filled with 180 µl/well of the buffer of the assay. Next, the donor plate was superimposed on the acceptor plate forming a "sandwich" for 2.5 hours at room temperature in a humid atmosphere. After the incubation period, the donor plate was removed and the absorbance of the solutions of the acceptor plate was read at the wavelengths previously established, calculating the effective permeability (*Ep*) of each compound based on the correlation established between the experimental *Ep* and the *Ep* described in the bibliography of the 10 control drugs according to the protocol described in the literature mentioned above **(Table 1)**. In this way, the probability of crossing the BBB of each study compound is predicted, being high (CNS +), low (CNS -) or uncertain (CNS +/ CNS -) **(Table 1).** Each sample was analysed from 3 to 5 wavelengths, in triplicate, and in 2 independent assays. The results are shown as the average of the 2 trials with their standard deviation value.

**Table 1. Data on enzyme inhibition and permeability of the blood-brain barrier (BBB) of the compounds of the invention. Prediction of the tested compounds crossing the BBB: high probability (CNS+), low (CNS -) and uncertain (CNS +/ CNS -); n.d.: not possible to determine under experimental conditions.**

| **Compound** | **TTBK1 IC₅₀ µM** | **TTBK2 IC₅₀ µM** | ***Ep* (10⁻⁶ cm s⁻¹)** | **Prediction** |
|---|---|---|---|---|
| 1 | 0.39±0.02 | 0.85±0.31 | 23.2±1.4 | CNS₊ |
| 2 | 0.24±0.1 | 4.22±1.55 | 11.5±1.0 | CNS₊ |
| 3 | 0.77±0.14 | 3.02±0.82 | 15.6±1.3 | CNS₊ |
| 4 | 0.44±0.31 | 2.14±1.2 | n.d. | n.d. |
| 5 | 0.54±0.53 | 0.97±0.5 | 11.8±0.3 | CNS₊ |
| 6 | 5.03±2.9 | 5% | 7.6±1.4 | CNS₊ |
| 7 | 0.75±0.03 | 1.21±0.06 | 11±1.2 | CNS + |
| 8 | 0.42±0.11 | 1.24±0.30 | 14.3±1.1 | CNS + |
| 9 | 0.52±0.08 | 4.90±1.21 | n.d. | n.d. |
| 10 | 1.02±0.33 | 8.79±2.00 | 18.7±1.4 | CNS + |
| 11 | 2.2±0.50 | 6.4±0.5 | 14±0.4 | CNS + |
| 12 | 1.55±1.84 | >100 | 8.4±1.6 | CNS + |
| 13 | 5.61±2.3 | 1 % | 6.8±1.4 | CNS + |
| 14 | 0.53±0.02 | 0.49±0.05 | 9.7±1.0 | CNS + |
| 15 | 3.56±1.63 | 9.54±0.09 | n.d. | n.d. |
| 16 | 2.30±0.54 | 5.72±2.65 | 17.3±0.1 | CNS + |
| 17 | 0.84±0.13 | 6.79±0.27 | 10.5±0.7 | CNS + |
| 18 | 0.58±0.08 | 4.49±1.16 | 7.5±0.1 | CNS + |
| 19 | 1.32±0.04 | 3.90±0.48 | 7.7±0.6 | CNS + |
| 20 | 0.45±0.04 | 2.70±0.29 | 9.7±1.0 | CNS + |
| 21 | 0.37±0.12 | 3.05±0.07 | 14.1±0.4 | CNS + |
| 22 | 2.72±1.78 | 22.72±2.84 | n.d. | n.d. |
| 23 | 0.65±0.05 | 4.94±0.28 | 4.7±0.5 | CNS + |
| 24 | 0.75±0.04 | 1.21±0.15 | 18.3±1.2 | CNS + |
| 25 | 1.71±0.87 | 11.92±4.17 | 6.9±0.6 | CNS + |
| 26 | 1.5±0.3 | 5.4±0.7 | 9.4±0.4 | CNS + |

| | | | | |
|---|---|---|---|---|
| **I.C.₅₀** refers to the inhibitory concentration necessary to inhibit 50% of the kinase activity. **Ep** refers to effective permeability of the blood-brain barrier (BBB) of the compounds | | | | |

### Example 4. Neuroprotection against ethacrynic acid

Human neuroblastoma cells (SH-SY5Y) are seeded in 96-well plates at a density of 60,000 cells/well. When they are at confluence, they are exposed to 40 µM of ethacrynic acid (EA), having been pre-treated one hour before with the study compounds and controls dissolved in DMSO at the determined concentration (5 µM). Cell viability was evaluated after an incubation period of 24 hours from the addition of the EA using the MTT assay (Morgan DM. Tetrazolium (MTT) assay for cellular viability and activity. Methods Mol Biol. 1998;79:179-183) **(****Figure 1****).**

### Example 5. Decreased TDP-43 phosphorylation

The evaluation of the neuroprotective effect at the protein expression level was analysed using the Western blot technique **(****Figure 2****).** Cells were seeded in 6-well plates at a density of 2×10⁶ cells per well. The experiment was carried out following the same protocol detailed in the previous section. After the incubation period in the presence or absence of the compounds, the medium is removed, washing is performed with PBS 1X, the cells are collected and 30 µl of lysis buffer (50 mM Tris pH 7.4, 1% Nonidet-40, 150 mM NaCl and 10 µl/ml of protease and phosphatase inhibitors) are added. After a freezethaw cycle at -80°C, the lysates are centrifuged at 15,000 rpm for 20 min, the protein fraction remaining in the supernatant.

Protein quantification was performed using the commercial Pierce^{™} BCA protein quantification kit. The separation of proteins based on their molecular weight, was performed by electrophoresis in 10% polyacrylamide gels, loading between 50 µg per sample. Transfer was performed to a PVDF (Polyvinylidene Fluoride) membrane at 4°C. Membranes were blocked with 5% bovine serum albumin or BSA dissolved in TBS-T buffer (50 mM Tris·HCl, pH 7.5, 150 mM NaCl, 0.1% Tween-20) for one hour. Incubation with primary antibodies (p-Ser409/410-TDP43 (1:800) TDP-43 (1:1000) from Proteintech and GAPDH (1:1000) from Santa cruz biotechnology) was performed overnight at 4°C. Secondary antibodies conjugated to horseradish peroxidase, or HRP, were incubated for one hour at room temperature after having performed 3 5-min washes with TBS-T. The secondary antibody signal was amplified by the ECL^{™} (Thermo Scientific) chemiluminescence detection system. The bands were quantified with the ChemiDoc (Bio-Rad) kit. The GAPDH protein was used as a loading control.

### Example 6. Neuroprotection against okadaic acid

Human neuroblastoma cells (SH-SY5Y) are seeded in 96-well plates at a density of 15,000 cells/well. When they are at confluence, they are exposed to 30 nM of okadaic acid (OA), having been pre-treated one hour before with the study compounds and controls dissolved in DMSO at the determined concentration (1 µM). Okadaic acid is a phosphatase inhibitor and its cellular toxicity is associated with an increase in hyperphosphorylation of the tau protein (Harris K.A., Oyler GA, Doolittle GM, Vincent I, Lehman RA, Kincaid RL, Billingsley ML. Okadaic acid induces hyperphosphorylated forms of tau protein in human brain slices. Ann Neurol. 1993 Jan;33(1):77-87). The neuroprotection carried out by the compounds was evaluated after an incubation period of 24 hours from the addition of the OA using the MTT assay (Morgan DM. Tetrazolium (MTT) assay for cellular viability and activity. Methods Mol Biol. 1998;79:179-183) **(****Figure 3****).**

### Conclusions

TTBK1 inhibitors, permeable to the blood-brain barrier and capable of reducing the phosphorylation of tau and TDP-43 in cell models, they may be good drug candidates for the treatment of diseases of the central nervous system that cause hyperphosphorylation of tau and TDP-43 proteins. To that end, the inhibitors described in the present patent could be used for the treatment and/or prevention of pathologies such as Alzheimer's disease, amyotrophic lateral sclerosis and frontotemporal dementia, in which half of the patients show alterations in tau and the other half in TDP-43, among other neurodegenerative pathologies.

## Claims

1. A compound of formula (I), any of the isomers thereof or the pharmaceutically acceptable salts thereof 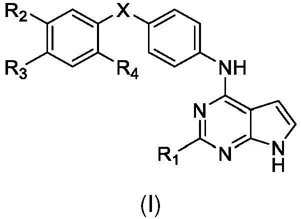 wherein
R₁ is selected from H and NH₂,
X is selected from O, S, CO and CH₂O,
R₂, R₃ and R₄ are independently selected from: H, halogen, CF₃, CN, NO₂, NH₂, C₁-C₃ alkyl and -O-C₁-C₃ alkyl,
and wherein at least one of R₂, R₃ and R₄ is H
for use in the treatment and/or prevention of tauopathies and/or TDP-43 diseases.

2. The compound for use, according to claim 1, wherein at least two of R₂, R₃ and R₄ are H.

3. The compound for use, according to any of claims 1 or 2, wherein R₁ is H.

4. The compound for use, according to any of claims 1 to 3, wherein X is O.

5. The compound for use, according to claim 1, wherein R₁ is H and X is O and wherein R₂, R₃ and R₄ are independently selected from H, halogen, CN, NO₂, NH₂, C₁-C₃ alkyl and -O-C₁-C₃ alkyl.

6. The compound for use, according to claim 5, wherein R₂ and R₄ are H and R₃ is selected from H, halogen, CN, NO₂, NH₂, C₁-C₃ alkyl and -O-C₁-C₃ alkyl.

7. The compound for use, according to claim 5, wherein R₂ and R₃ are H and R₄ is selected from halogen and -O-C₁-C₃ alkyl.

8. The compound for use, according to claim 5, wherein R₃ and R₄ are H and R₂ is selected from halogen, -(O)-C₁-C₃ alkyl and C₁-C₃ alkyl.

9. The compound for use, according to any of the preceding claims 1 to 3, wherein X is S.

10. The compound for use, according to claim 9, wherein R₁ is H, and R₂, R₃ and R₄ are independently selected from H, NO₂ and NH₂.

11. The compound for use, according to any of the preceding claims 1 to 3, wherein X is CO.

12. The compound for use, according to claim 11, wherein R₁ is H and R₂, R₃ and R₄ are independently selected from halogen and H.

13. The compound for use, according to any of the preceding claims 1 to 2, wherein R₁ is NH₂.

14. The compound for use, according to claim 13, wherein X is O.

15. The compound for use, according to claim 14, wherein R₂, R₃ and R₄ are independently selected from: H, halogen and CF₃, at least one of them being different from H.

16. The compound for use, according to claim 1 wherein the compound of formula (I) is selected from the list comprising: *N*-(4-phenoxyphenyl)-*7H*-pyrrolo[2,3-*d*]-pyrimidin-4-amine *N*-[4-(4-chlorophenoxy)phenyl]-*7H*-pyrrolo[2,3-*d*]pyrimidin-4-amine *N*-[4-(4-fluorophenoxy)phenyl]-7*H*-pyrrolo[2,3-*d*]pyrimidin-4-amine *N*-[4-(4-cyanophenoxy)phenyl]-7*H*-pyrrolo[2,3-*d*]pyrimidin-4-amine *N*-[4-(4-methoxyphenoxy)phenyl]-*7H*-pyrrolo[2,3-*d*]pyrimidin-4-amine *N*-[4-(4-trifluoromethylphenoxy)phenyl]-*7H*-pyrrolo[2,3-*d*]pyrimidin-4-amine *N*-[4-(4-bromophenoxy)phenyl]-*7H*-pyrrolo[2,3-*d*]pyrimidin-4-amine *N*-[4-(4-nitrophenoxy)phenyl]-*7H*-pyrrolo[2,3-*d*]pyrimidin-4-amine *N*-[4-(4-aminophenoxy)phenyl]-*7H*-pyrrolo[2,3-*d*]pyrimidin-4-amine *N*-[4-(4-methylphenoxy)phenyl]-*7H*-pyrrolo[2,3-*d*]pyrimidin-4-amine *N*-[4-(3-methylphenoxy)phenyl]-*7H*-pyrrolo[2,3-*d*]pyrimidin-4-amine *N*-[4-(3-chlorophenoxy)phenyl]-*7H*-pyrrolo[2,3-*d*]pyrimidin-4-amine *N*-[4-(3-trifluoromethylphenoxy)phenyl]-*7H*-pyrrolo[2,3-*d*]pyrimidin-4-amine *N*-[4-(2-chlorophenoxy)phenyl]-*7H*-pyrrolo[2,3-*d*]pyrimidin-4-amine *N*-[4-(2-methoxyphenoxy)phenyl]-*7H*-pyrrolo[2,3-*d*]pyrimidin-4-amine *N*-[4-(2.4-chlorophenoxy)phenyl]-*7H*-pyrrolo[2,3-*d*]pyrimidin-4-amine *N*-[4-(3-methoxyphenoxy)phenyl]-*7H*-pyrrolo[2,3-*d*]pyrimidin-4-amine *N*4-(4-(4-(trifluoromethyl)phenoxy)phenyl]-*7H*-pyrrolo[2,3-*d*]pyrimidine-2,4-diamine *N*4-(4-(3-(trifluoromethyl)phenoxy)phenyl]-*7H*-pyrrolo[2,3-*d*]pyrimidine-2,4-diamine *N*4-(4-(4-chlorophenoxy)phenyl]-*7H*-pyrrolo[2,3-*d*]pyrimidine-2,4-diamine *N*4-(4-(3-chlorophenoxy)phenyl)-*7H*-pyrrolo[2,3-*d*]pyrimidine-2,4-diamine *N*-[4-((4-nitrophenyl)thio)phenyl]-*7H*-pyrrolo[2,3-*d*]pyrimidin-4-amine *N*-[4-((4-aminophenyl)thio)phenyl]-*7H*-pyrrolo[2,3-*d*]pyrimidin-4-amine *N*-[4-(benzyloxy)phenyl]-*7H*-pyrrolo[2,3-*d*]pyrimidin-4-amine (4-((*7H*-pyrrolo[2,3-*d*]pyrimidin-4-yl)amino)phenyl](phenyl)methanone (4-((*7H*-pyrrolo[2,3-*d*]pyrimidin-4-yl)amino)phenyl](4-fluorophenyl)methanone.

17. The compound for use, according to any of claims 1 to 16, wherein the tauopathy is selected from the following list: Alzheimer's disease (AD), progressive supranuclear palsy, frontotemporal dementia (FTD), Pick's disease, Down's syndrome.

18. The compound for use, according to any of claims 1 to 16, wherein the TDP-43 diseases are selected from the following list: amyotrophic lateral sclerosis (ALS), Alexander disease, limbic-predominant age-related TDP-43 encephalopathy (LATE), frontotemporal dementia (FTD) and Huntington's disease.

19. A compound of formula (I) 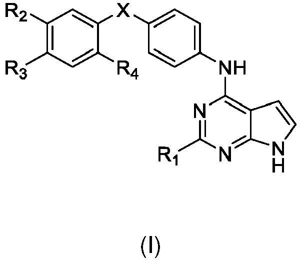 or any of the isomers thereof or the pharmaceutically acceptable salts thereof, wherein R₁, X, R₂, R₃ and R₄ have the same meaning described in any of claims 1 to 15 and wherein at least one of R₂, R₃ and R₄ are H, provided that the compound is neither of the following two:

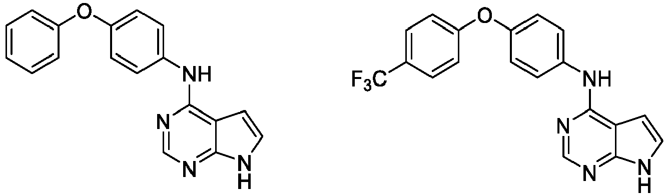

20. The compound according to claim 19, wherein the compound is selected from the following list: *N*-[4-(4-chlorophenoxy)phenyl]-*7H*-pyrrolo[2,3-*d*]pyrimidin-4-amine *N*-[*N*-[4-(4-fluorophenoxy)phenyl]-*7H*-pyrrolo[2,3-*d*]pyrimidin-4-amine *N*-[4-(4-cyanophenoxy)phenyl]-7*H*-pyrrolo[2,3-*d*]pyrimidin-4-amine *N*-[4-(4-methoxyphenoxy)phenyl]-*7H*-pyrrolo[2,3-*d*]pyrimidin-4-amine *N*-[4-(4-bromophenoxy)phenyl]-*7H*-pyrrolo[2,3-*d*]pyrimidin-4-amine *N*-[4-(4-nitrophenoxy)phenyl]-*7H*-pyrrolo[2,3-*d*]pyrimidin-4-amine *N*-[4-(4-aminophenoxy)phenyl]-*7H*-pyrrolo[2,3-*d*]pyrimidin-4-amine *N*-[4-(4-methylphenoxy)phenyl]-*7H*-pyrrolo[2,3-*d*]pyrimidin-4-amine *N*-[4-(3-methylphenoxy)phenyl]-*7H*-pyrrolo[2,3-*d*]pyrimidin-4-amine *N*-[4-(3-chlorophenoxy)phenyl]-*7H*-pyrrolo[2,3-*d*]pyrimidin-4-amine *N*-[4-(3-trifluoromethylphenoxy)phenyl]-*7H*-pyrrolo[2,3-*d*]pyrimidin-4-amine *N*-[4-(2-chlorophenoxy)phenyl]-*7H*-pyrrolo[2,3-*d*]pyrimidin-4-amine *N*-[4-(2-methoxyphenoxy)phenyl]-*7H*-pyrrolo[2,3-*d*]pyrimidin-4-amine *N*-[4-(2.4-chlorophenoxy)phenyl]-*7H*-pyrrolo[2,3-*d*]pyrimidin-4-amine *N*-[4-(3-methoxyphenoxy)phenyl]-*7H*-pyrrolo[2,3-*d*]pyrimidin-4-amine *N*4-(4-(4-(trifluoromethyl)phenoxy)phenyl]-*7H*-pyrrolo[2,3-*d*]pyrimidine-2,4-diamine *N*4-(4-(3-(trifluoromethyl)phenoxy)phenyl]-*7H*-pyrrolo[2,3-*d*]pyrimidine-2,4-diamine *N*4-(4-(4-chlorophenoxy)phenyl]-*7H*-pyrrolo[2,3-*d*]pyrimidine-2,4-diamine *N*4-(4-(3-chlorophenoxy)phenyl)-*7H*-pyrrolo[2,3-*d*]pyrimidine-2,4-diamine *N*-[4-((4-nitrophenyl)thio)phenyl]-*7H*-pyrrolo[2,3-*d*]pyrimidin-4-amine *N*-[4-((4-aminophenyl)thio)phenyl]-*7H*-pyrrolo[2,3-*d*]pyrimidin-4-amine *N*-[4-(benzyloxy)phenyl]-*7H*-pyrrolo[2,3-*d*]pyrimidin-4-amine (4-((*7H*-pyrrolo[2,3-*d*]pyrimidin-4-yl)amino)phenyl](phenyl)methanone (4-((*7H*-pyrrolo[2,3-*d*]pyrimidin-4-yl)amino)phenyl](4-fluorophenyl)methanone.

21. A compound of formula (I), any of the isomers thereof or the pharmaceutically acceptable salts thereof, according to any of claims 19 or 20, for use thereof as a medicinal product.

22. A pharmaceutical composition comprising a compound of formula (I), any of the isomers thereof or the pharmaceutically acceptable salts thereof, as defined in claim 19 or 20, and at least one excipient, pharmaceutically acceptable adjuvant and/or carrier.
